# EUROPEAN PATENT SPECIFICATION

(11) **EP 3 179 980 B1**
(45) Date of publication and mention of the grant of the patent: **04.12.2019**
(21) Application number: 15736508.1
(22) Date of filing: 13.07.2015
(51) Int. Cl.: A61K 8/46, A61Q 5/02, A61Q 5/12, A61K 8/73

(54) **SHAMPOO COMPOSITION**
SHAMPOOZUSAMMENSETZUNG
COMPOSITION DE SHAMPOING

(30) Priority: 13.08.2014 WO PCT/CN2014/084299; 16.10.2014 EP 14189215
(43) Date of publication of application: 21.06.2017
(73) Proprietor: Unilever NV, 3013 AL Rotterdam (NL); Unilever PLC, London, EC4Y 0DY (GB)
(72) Inventor: LI, Zhengrong, Shanghai 200335 (CN); PAN, Xuemiao, Shanghai 200335 (CN)
(74) Representative: Potter Clarkson
(86) International application number: PCT/EP2015/065939
(87) International publication number: WO 2016/023694

(56) References cited:
- EP-A1- 2 532 343
- CN-A- 102 579 307
- DATABASE GNPD [Online] MINTEL; 28 February 2013 (2013-02-28), "Shampoo", XP002737802, Database accession no. 1994626

## Description

### Field of the invention

The present invention relates to shampoo compositions. In particular, the invention relates to shampoo compositions, which are substantially free of silicone conditioning agents.

### Background of the invention

Consumers are increasingly looking for shampoo products that provide more than cleansing of the hair fibres. In particular, consumers often desire cleansing that does not result in unmanageable hair. Thus, shampoos have been developed which simultaneously clean and condition the hair.

Most conventional conditioning shampoos contain silicone polymers which are deposited onto the hair fibres during rinsing. US 6,180,576 discloses one such conditioning shampoo composition that contains a dispersed, insoluble, nonionic silicone hair conditioning agent.

The use of insoluble silicone hair conditioning agents provides opacity to shampoo compositions that may not always be desirable.

Previous efforts have been made to provide compositions that allow for clearer shampoos to be formulated. WO 2004/105710 A discloses a conditioning shampoo comprising: (a) 8-18 weight percent of an anionic surfactant; (b) 0.5-0.8 weight percent of a conditioning system comprising: (i) 0.1-0.75 weight percent of silicone Quaternium-8; (ii) 0.1-0.5 weight percent of a low molecular weight guar gum with a molecular weight less than 100,000 centipoise as an aqueous clear cationic solution of a modified polysaccharide clear cationic solution; and (iii) 0.1-0.5 weight percent of Polyquaternium-10; (c) 1.0-6.0 weight percent of an amphoteric surfactant; (d) 0.5-5.0 weight percent of a member selected from the group consisting of cocodiethanol amide, and cocomonoethanol amide; and (e) the remainder as water. This shampoo comprises a water soluble silicone (the silicone Quaternium-8).

The present inventors have recognized that some consumers have a negative perception of silicones and seek silicone-free hair care compositions. Thus, the inventors have identified a need for shampoos that provide good cleansing, conditioning and which are substantially free from silicones. The inventors have also recognized that compositions are desirable which provide the foregoing benefits and, at least in some embodiments, allow for formulating less opaque or even transparent shampoos.

### Summary of the invention

In one aspect, the present invention is directed to a shampoo composition comprising:
a) from 3 to 11 wt% alkyl sulfate surfactant, ethoxylated alkyl sulfate surfactant or mixture thereof;
b) from 1 to 7 wt% fatty acyl isethionate; and
c) from 0.01 to 2 wt% cationic polymer, wherein the cationic polymer comprises cationic galactomannan and cationic cellulose;
d) from 0 to 0.1 wt% silicone conditioning agent;
wherein the weight ratio of cationic galactomannan to cationic cellulose in the composition is in the range of from 1:1 to 10:1.

Preferably the shampoo composition is free of silicone conditioning agent. All other aspects of the present invention will more readily become apparent upon considering the detailed description and examples which follow.

### Detailed description

The shampoo composition comprises an alkyl sulfate and/or ethoxylated alkyl sulfate anionic surfactant at a level of from 3 to 11 wt.%, more preferably from 5 to 10 wt.%. The alkyl sulfate and/or ethoxylated alkyl sulfate provides the shampoo with excellent cleansing properties. Thus, it is preferred that the total amount of alkyl sulfate and ethoxylated alkyl sulfate is at least 3% by weight of the shampoo composition, more preferably at least 5%, and most preferably at least 6%.

The amount of alkyl sulfate and/or ethoxylated alkyl sulfate is not too high in order to avoid negative effects on conditioning performance of the shampoo. Thus it is preferred that the total amount of alkyl sulfate and ethoxylated alkyl sulfate is no more than 11% by weight of the shampoo composition, more preferably no more than 10%, and most preferably no more than 8%.

Preferred alkyl sulfates are C₈₋₁₈ alky sulfates, more preferably C₁₂₋₁₈ alkyl sulfates, preferably in the form of a salt with a solubilising cation such as sodium, potassium, ammonium or substituted ammonium. Examples are sodium lauryl sulfate (SLS) or sodium dodecyl sulfate (SDS).

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES).

Most preferred is ethoxylated alkyl sulfate anionic surfactant is sodium lauryl ether sulfate (SLES) having an average degree of ethoxylation of from 0.5 to 3, preferably 1 to 3.

The fatty acyl isethionate is present in the shampoo composition at a level of from 1 to 7 wt.%.

The fatty acyl isethionate contributes to both the cleansing and conditioning properties of the shampoo. It is preferred that the shampoo comprises the fatty acyl isethionate in an amount of at least 1% by weight of the shampoo composition, more preferably at least 1.5% and most preferably at least 2%.

The amount of fatty acyl isethionate need not be too high as cleaning performance is also provided by the alkyl sulfate and/or ethoxylated alkyl sulfate and conditioning is aided by the specific cationic polymer system of the invention. Thus it is preferred that the total amount of fatty acyl isethionate is no more than 7% by weight of the shampoo composition, more preferably no more than 6%, and most preferably no more than 5%.

Prefered fatty acyl isethionates are those with a fatty chain length greater than or equal to C₆. More proffered are fatty acyl isethionates with a fatty chain length greater than or equal to C₇, even more preferably with a fatty chain length of from C₈ to C₁₆. Most preferably the fatty acyl isethionate comprises at least 50% by weight of the fatty acyl isethionate of material with a chain length of C₁₂ (e.g., lauroyl isethionate), more preferably at least 60%, more preferably 70%, and most preferably 80 to 100%.

Preferably, the fatty acyl isethionate is in the form of a salt with a solubilising cation such as sodium, potassium, ammonium or substituted ammonium, more preferably sodium. The most preferred fatty acyl isethionate is Sodium Lauroyl Isethionate.

In addition to the alkl sulfate, ethoxylated alkyl sulfate and fatty acyl isethionate, shampoo compositions according to the invention may comprise one or more further anionic surfactants which are cosmetically acceptable and suitable for topical application to the hair.

Examples of further suitable anionic surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical further anionic cleansing surfactants for use in shampoo compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate (n)EO, (where n is from 1 to 3), lauryl ether carboxylic acid (n) EO (where n is from 10 to 20).

Mixtures of any of the foregoing anionic cleansing surfactants may also be suitable.

Preferably the alkly sulfate, ethoxylated alkyl sulfate and fatty acyl isethionate collectively account for at least 70% by weight of total anionic surfactant in the composition, more preferably at least 80%, more preferably still from 90 to 100%.

It has also been found beneficial to the sensory properties of shampoo compositions of the present invention to include, in some embodiments, free fatty acid and/or fatty acid salt. Preferred free fatty acid/salts are those with chain length C₁₆ to C₂₀. Especially preferred is stearic acid and/or its salts.

Preferably, the shampoo composition comprises the fatty acid and/or its salt in a total amount of from 0.001 to 5% by weight of the shampoo composition, more preferably from 0.01 to 3%, more preferably still from 0.05 to 2% and most preferably from 0.1 to 1%.

The composition can include co-surfactants, to help impart aesthetic, physical or cleansing properties to the composition. Co-surfactants are typically selected from nonionic surfactant, amphoteric surfactant, zwitterionic surfactants and mixtures thereof.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred co-surfactant surfactant is betaine surfactant, especially alkyl amidopropyl betaine such as cocamidopropyl betaine (CAPB).

Co-surfactant can be included, for example, in an amount ranging from 0.1 to 10%, more preferably from 1 to 5%, most preferably from 1.5 to 4% by weight based on the total weight of the composition.

The shampoo compositions of the present invention have excellent conditioning performance even in the absence of silicone conditioning agents. The terms "silicones" and "silicone conditionings agents" are used interchangeably herein and refer to compounds comprising at least one silicon atom. The shampoo composition is substantially free of silicone conditioning agent.

As used herein, the term "substantially free of" a substance means that the shampoo composition comprises from 0 to 0.1% of the substance by weight of the shampoo composition, more preferably comprises less than 0.05%, even more preferably less than 0.01%, still more preferably less than 0.001% and most preferably comprises 0%.

As well as conditioning provided by the fatty acyl isethionate, the shampoo compositions of the present invention benefit from conditioning provided by a specific cationic polymer system. The cationic polymer for use in the present invention comprises both cationic galactomannan and cationic cellulose.

Cationic cellulose is available, for example, from the Dow Chemical Company in their UCARE JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Dow Chemical Company under the tradename Polymer LM-200. Most preferred is Polyquaternium 10, especially Polyquaternium 10 with a molecular weight (weight average by GPC) in the range 0.5 to 3 million Daltons and charge density in the range 0.5 to 3 meq/g. The preferred Polyquaternium 10 is available, for example, as UCARE™ JR30M from Dow Chemical.

Cationic galactomannans include cationic guar derivatives and cationic cassia derivatives. Most preferred are cationic guar derivatives, especially Guar Hydroxypropyltrimonium Chloride. The latter is available, for example, under the tradename JAGUAR from Rhodia. Especially preferred is cationic guar with a molecular weight (weight average by GPC) of from 0.2 to 1.5 million Daltons and a charge density of from 0.5 to 0.9 meq/g. Such a polymer is available, for example, as JAUGUAR EXCEL™ from Rhodia.

Molecular weights and charge densities may be determined using GPC (also known as SEC) and NMR respectively as described, for example, in WO 2013/011122 A.

In addition to the cationic cellulose and cationic galactomannan, the cationic polymer in the shampoo composition may comprise further cationic conditioning polymers. Suitable further cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The total amount of cationic polymer in the shampoo composition is from 0.01 to 2 wt%. Preferably, the shampoo composition comprises cationic polymer in a total amount of from 0.05 to 1.5% by weight of the shampoo composition, more preferably from 0.1 to 1% and most preferably from 0.2 to 0.7%. Preferably the cationic galactomannan and cationic cellulose together amount for at least 50% by weight of the total cationic polymer in the shampoo composition, more preferably at least 70%, more preferably still at least 80% and most preferably from 90 to 100%.

The amount of cationic galactomannan in the composition is preferably in the range 0.05 to 0.6% by weight of the shampoo composition, more preferably from 0.1 to 0.5% and most preferably from 0.15 to 0.4%.

The amount of cationic cellulose in the composition is preferably in the range 0.01 to 0.4% by weight of the shampoo composition, more preferably from 0.05 to 0.3% and most preferably from 0.07 to 0.2%.

The weight ratio of cationic galactomannan to cationic cellulose in the composition is in the range from 1:1 to 10:1, more preferably 1:1.5 to 4:1, even more preferably 1:1 to 3:1, and most preferably 1.1:1 to 2:1.

Shampoo compositions of the invention are preferably aqueous, i.e. they have water or an aqueous solution or a lyotropic liquid crystalline phase as their major component. Suitably, the composition will comprise from 50 to 98%, more preferably from 60 to 90%, and most preferably from 75 to 87% water by weight based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

In a preferred embodiment, the shampoo composition of the invention is transparent or at least translucent. In particular it is preferred that the composition has a transmittance at 800 nm (path length of 1 cm and temperature of 25°C) of at least 1%, more preferably at least 10%, more preferably still at least 40%, even more preferably at least 70% and most preferably from 80 to 100%.

Except in the examples, or where otherwise explicitly indicated, all numbers in this description indicating amounts of material or conditions of reaction, physical properties of materials and/or use may optionally be understood as modified by the word "about". All amounts are by weight of the final shampoo composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Examples

### Example 1

This example demonstrates the improved conditioning performance of a shampoo according to the invention as determined by texture analysis.

Two shampoos were made with the formulations shown in table 1 (amounts are in terms of amount of active ingredient by weight of the total shampoo composition).

**TABLE 1**

| Ingredient | Shampoo 1 | Shampoo A |
|---|---|---|
| SLES | 8.0 | 12 |
| Sodium Lauroyl Isethionate | 3.2 | --- |
| Stearic Acid | 0.0001 | 0.0001 |
| CAPB | 3.0 | 1.6 |
| Guar Hydroxypropyltrimonium Chloride¹ | 0.25 | 0.25 |
| Polyquaternium 10² | 0.15 | 0.15 |
| Salt, Perfume and Preservatives | <2% | <2% |
| Water | To 100 | To 100 |

| | | |
|---|---|---|
| 1. Jaguar Excel™ from Rhodia. 2. UCARE™ JR30M. | | |

Hair switches were washed with one of the two shampoos in an identical manner, treated with an identical conditioner, rinsed and then dried. Texture analysis of the hair switches was then performed using a TA.XT plus Texture Analyser. The mean friction measured for 5 repeat measurements for each sample is reported in Table 2 along with the standard deviation in parentheses. Lower friction indicates better conditioning.

**TABLE 2**

| Shampoo | Friction (Nm) |
|---|---|
| 1 | 40.1 (5.0) |
| A | 48.8 (1.6) |

### Example 2

This example demonstrates the improved performance of a shampoo according to the invention as determined by a sensory study.

A total of 36 panelists had their hair washed in consistent manner by trained stylists. Half the participants had their hair washed with Sampoo A from Example 1 and the remaining half with Shampoo 2 shown in Table 3 below.

**TABLE 3**

| Ingredient | Shampoo 2 |
|---|---|
| SLES | 8.0 |
| Sodium Lauroyl Isethionate | 3.2 |
| Stearic Acid | 0.5 |
| CAPB | 3.0 |
| Guar Hydroxypropyltrimonium Chloride³ | 0.25 |
| Polyquaternium 10⁴ | 0.15 |
| Salt, Perfume and Preservatives | <2% |
| Water | To 100 |

| | |
|---|---|
| 3. Jaguar Excel™ from Rhodia. 4. UCARE™ JR30M. | |

The stylists were asked to evaluate the panelists' hair against a range of standard attributes. The results showed that Shampoo 2 was significantly (at least 95% conf.) higher in the following attributes:

### Shampooing Stage:

- Longer time to rinse strip
- Easier to fingering through during rinse
- More slippery feel after rinse (under running water)

### Wet Stage:

- Easier wet combing - Total effort
- Easier wet combing - single stroke
- More slippery feel wet
- More coating wet

### Dry Stage:

- Easier dry combing-Total effort
- Easier dry combing- single stroke
- Less stick out
- More slippery feel
- Hair surface smoother
- More coating
- Finger through
- More weighty hair
- More sticky

The results showed that Shampoo A was significantly (at least 95% conf.) higher in the following attributes:

### Dry Stage:

- Volume
- More visual expansion from scalp
- More shiny
- More elastic when compressed
- Higher hair dryness
- Static

These results showed that Shampoo 2 provided significantly better conditioning in both wet and dry stage than Shampoo A.

## Claims

1. A shampoo composition comprising:
a) from 3 to 11 wt% alkyl sulfate surfactant, ethoxylated alkyl sulfate surfactant or mixture thereof;
b) from 1 to 7 wt% fatty acyl isethionate;
c) from 0.01 to 2 wt% cationic polymer, wherein the cationic polymer comprises cationic galactomannan and cationic cellulose; and
d) from 0 to 0.1 wt% silicone conditioning agent;
wherein the weight ratio of cationic galactomannan to cationic cellulose in the composition is in the range from 1:1 to 10:1.

2. The shampoo composition as claimed in claim 1 wherein the composition comprises from 0.01 to 5 wt% free fatty acid and/or fatty acid salt.

3. The shampoo composition as claimed in claim 2 wherein the fatty acid and/or fatty acid salt comprises stearic acid and/or a salt thereof

4. The shampoo composition as claimed in any one of the preceding claims wherein the amount of silicone is less than 0.05% by weight of the composition, preferably less than 0.01%.

5. The shampoo composition as claimed in any one of the preceding claims wherein the amount of alkyl sulfate surfactant, ethoxylated alkyl sulfate surfactant or mixture thereof is from 5 to 10 wt%.

6. The shampoo composition as claimed in any one of the preceding claims wherein the amount of fatty acyl isethionate is from 1.5 to 5% by weight of the composition.

7. The shampoo composition as claimed in any one of the preceding claims wherein the amount of cationic galactomannan is from 0.05 to 0.5% by weight of the composition, preferably from 0.1 to 0.4%.

8. The shampoo composition as claimed in any one of the preceding claims wherein the amount of cationic cellulose is from 0.01 to 0.4% by weight of the composition, preferably from 0.05 to 0.3%.

9. The shampoo composition as claimed in any one of the preceding claims wherein the weight ratio of cationic galactomannan to cationic cellulose is from 1:1 to 4:1.

10. The shampoo composition as claimed in any one of the preceding claims wherein the shampoo comprises betaine surfactant, preferably alkyl amidopropyl betaine.

11. The shampoo composition as claimed in claim 10 wherein the shampoo comprises from 0.1 to 10 wt% of the betaine surfactant, preferably from 1 to 5 wt%.

12. The shampoo composition as claimed in any one of the preceding claims wherein the fatty acyl isethionate comprises or is Lauroyl Isethionate.

13. The shampoo composition as claimed in any one of the preceding claims comprising:
a) from 5 to 11 wt% alkyl sulfate surfactant, ethoxylated alkyl sulfate surfactant or mixture thereof;
b) from 1.5 to 5 wt% fatty acyl isethionate;
c) from 0.15 to 0.7 wt% cationic polymer, wherein the cationic polymer comprises:
(i) from 0.1 to 0.4% cationic galactomannan by weight of the shampoo composition; and
(j) from 0.05 to 0.3% cationic cellulose by weight of the shampoo composition; and
d) from 0 to 0.05 wt% silicone conditioning agent.

14. The shampoo composition as claimed in any one of the preceding claims wherein the cationic cellulose is Polyquaternium 10.

15. The shampoo composition as claimed in any one of the preceding claims wherein the cationic galactomannan is Guar Hydroxypropyltrimonium Chloride.

## Patentansprüche

1. Shampoozusammensetzung, umfassend:
a) von 3 bis 11 Gew.-% Alkylsulfat-Tensid, ethoxyliertes Alkylsulfat-Tensid oder eine Mischung davon;
b) von 1 bis 7 Gew.-% Fettacylisethionat;
c) von 0,01 bis 2 Gew.-% kationisches Polymer, wobei das kationische Polymer kationisches Galactomannan und kationische Cellulose umfasst; und
d) von 0 bis 0,1 Gew.-% Silikon-Konditionierungsmittel,
wobei das Gewichtsverhältnis von kationischem Galactomannan zu kationischer Cellulose in der Zusammensetzung in dem Bereich von 1:1 bis 10:1 liegt.

2. Shampoozusammensetzung, wie im Anspruch 1 beansprucht, wobei die Zusammensetzung von 0,01 bis 5 Gew.-% freie Fettsäure und/oder Fettsäuresalz umfasst.

3. Shampoozusammensetzung, wie im Anspruch 2 beansprucht, wobei die Fettsäure und/oder das Fettsäuresalz Stearinsäure und/oder ein Salz davon umfassen.

4. Shampoozusammensetzung, wie in irgendeinem der vorhergehenden Ansprüche beansprucht, wobei die Menge des Silikons weniger als 0,05 Gewichts-% der Zusammensetzung, vorzugsweise weniger als 0,01 %, beträgt.

5. Shampoozusammensetzung, wie in irgendeinem der vorhergehenden Ansprüche beansprucht, wobei die Menge des Alkylsulfat-Tensids, ethoxylierten Alkylsulfat-Tensids oder der Mischung davon von 5 bis 10 Gew.-% beträgt.

6. Shampoozusammensetzung, wie in irgendeinem der vorhergehenden Ansprüche beansprucht, wobei die Menge des Fettacylisethionats von 1,5 bis 5 Gewichts-% der Zusammensetzung beträgt.

7. Shampoozusammensetzung, wie in irgendeinem der vorhergehenden Ansprüche beansprucht, wobei die Menge des kationischen Galactomannans von 0,05 bis 0,5 Gewichts-% der Zusammensetzung, vorzugsweise von 0,1 bis 0,4 %, beträgt.

8. Shampoozusammensetzung, wie in irgendeinem der vorhergehenden Ansprüche beansprucht, wobei die Menge der kationischen Cellulose von 0,01 bis 0,4 Gewichts-% der Zusammensetzung, vorzugsweise von 0,05 bis 0,3 %, beträgt.

9. Shampoozusammensetzung, wie in irgendeinem der vorhergehenden Ansprüche beansprucht, wobei das Gewichtsverhältnis von kationischem Galactomannan zu kationischer Cellulose von 1:1 bis 4:1 beträgt.

10. Shampoozusammensetzung, wie in irgendeinem der vorhergehenden Ansprüche beansprucht, wobei das Shampoo Betain-Tensid, vorzugsweise Alkylamidopropylbetain, umfasst.

11. Shampoozusammensetzung, wie im Anspruch 10 beansprucht, wobei das Shampoo von 0,1 bis 10 Gew.-% Betain-Tensid, vorzugsweise von 1 bis 5 Gew.-%, umfasst.

12. Shampoozusammensetzung, wie in irgendeinem der vorhergehenden Ansprüche beansprucht, wobei das Fettacylisethionat Lauorylisethionat umfasst oder ist.

13. Shampoozusammensetzung, wie in irgendeinem der vorhergehenden Ansprüche beansprucht, umfassend:
a) von 5 bis 11 Gew.-% Alkylsulfat-Tensid, ethoxyliertes Alkylsulfat-Tensid oder eine Mischung davon;
b) von 1,5 bis 5 Gew.-% Fettacylisethionat;
c) von 0,15 bis 0,7 Gew.-% kationisches Polymer, wobei das kationische Polymer umfasst:
(i) von 0,1 bis 0,4 % kationisches Galactomannan, bezogen auf das Gewicht der Shampoozusammensetzung; und
(j) von 0,05 bis 0,3 % kationische Cellulose, bezogen auf das Gewicht der Shampoozusammensetzung; und
d) von 0 bis 0,05 Gew.-% Silikon-Konditionierungsmittel.

14. Shampoozusammensetzung, wie in irgendeinem der vorhergehenden Ansprüche beansprucht, wobei die kationische Cellulose Polyquaternium 10 ist.

15. Shampoozusammensetzung, wie in irgendeinem der vorhergehenden Ansprüche beansprucht, wobei das kationische Galactomannan GuarHydroxypropyltrimoniumchlorid ist.

## Revendications

1. Composition de shampoing comprenant :
a) de 3 à 11 % en masse de tensioactif de sulfate d'alkyle, tensioactif de sulfate d'alkyle éthoxylé ou mélange de ceux-ci ;
b) de 1 à 7 % en masse d'iséthionate d'acyle gras ;
c) de 0,01 à 2 % en masse de polymère cationique, dans laquelle le polymère cationique comprend du galactomannane cationique et de la cellulose cationique ; et
d) de 0 à 0,1 % en masse d'agent conditionnant de silicone ;
dans laquelle le rapport massique de galactomannane cationique à cellulose cationique dans la composition se trouve dans l'intervalle de 1:1 à 10:1.

2. Composition de shampoing selon la revendication 1, dans laquelle la composition comprend de 0,01 à 5 % en masse d'acide gras et/ou sel d'acide gras libre.

3. Composition de shampoing selon la revendication 2, dans laquelle l'acide gras et/ou sel d'acide gras comprend de l'acide stéarique et/ou un sel de celui-ci.

4. Composition de shampoing selon l'une quelconque des revendications précédentes, dans laquelle la quantité de silicone est inférieure à 0,05 % en masse de la composition, de préférence inférieure à 0,01 %.

5. Composition de shampoing selon l'une quelconque des revendications précédentes, dans laquelle la quantité de tensioactif de sulfate d'alkyle, tensioactif de sulfate d'alkyle éthoxylé ou mélange de ceux-ci est de 5 à 10 % en masse.

6. Composition de shampoing selon l'une quelconque des revendications précédentes, dans laquelle la quantité d'iséthionate d'acyle gras est de 1,5 à 5 % en masse de la composition.

7. Composition de shampoing selon l'une quelconque des revendications précédentes, dans laquelle la quantité de galactomannane cationique est de 0,05 à 0,5 % en masse de la composition, de préférence de 0,1 à 0,4 %.

8. Composition de shampoing selon l'une quelconque des revendications précédentes, dans laquelle la quantité de cellulose cationique est de 0,01 à 0,4 % en masse de la composition, de préférence de 0,05 à 0,3 %.

9. Composition de shampoing selon l'une quelconque des revendications précédentes, dans laquelle le rapport massique de galactomannane cationique à cellulose cationique est de 1:1 à 4:1.

10. Composition de shampoing selon l'une quelconque des revendications précédentes, dans laquelle le shampoing comprend un tensioactif de bétaïne, de préférence une alkylamidopropylbétaïne.

11. Composition de shampoing selon la revendication 10, dans laquelle le shampoing comprend de 0,1 à 10 % en masse du tensioactif de bétaïne, de préférence de 1 à 5 % en masse.

12. Composition de shampoing selon l'une quelconque des revendications précédentes, dans laquelle l'iséthionate d'acyle gras comprend ou est l'iséthionate de lauroyle.

13. Composition de shampoing selon l'une quelconque des revendications précédentes comprenant :
a) de 5 à 11 % en masse de tensioactif de sulfate d'alkyle, tensioactif de sulfate d'alkyle éthoxylé ou mélange de ceux-ci ;
b) de 1,5 à 5 % en masse d'iséthionate d'acyle gras ;
c) de 0,15 à 0,7 % en masse de polymère cationique, dans laquelle le polymère cationique comprend :
(i) de 0,1 à 0,4 % de galactomannane cationique en masse de la composition de shampoing ; et
(j) de 0,05 à 0,3 % de cellulose cationique en masse de la composition de shampoing ; et
d) de 0 à 0,05 % en masse d'agent conditionnant de silicone.

14. Composition de shampoing selon l'une quelconque des revendications précédentes, dans laquelle la cellulose cationique est Polyquaternium 10.

15. Composition de shampoing selon l'une quelconque des revendications précédentes, dans laquelle le galactomannane cationique est le chlorure de guar hydroxypropyltrimonium.
